# EUROPEAN PATENT SPECIFICATION

(11) **EP 1 567 474 B1**
(45) Date of publication and mention of the grant of the patent: **22.10.2008**
(21) Application number: 03812156.2
(22) Date of filing: 24.11.2003
(51) Int. Cl.: C07C 69/00, C07C 67/48, C07C 67/58

(54) **PROCESS FOR PURIFYING DIACEREIN**
VERFAHREN ZUR AUFREINIGUNG VON DIACEREIN
PROCEDE DE PURIFICATION DE DIACEREINE

(30) Priority: 29.11.2002 IT MI20022535
(43) Date of publication of application: 31.08.2005
(73) Proprietor: Synteco S.p.A., 27028 S. Martino Siccomario (IT)
(72) Inventor: MAGGI, Domenico, I-27028 San Martino Siccomario (IT)
(74) Representative: Bianchetti, Giuseppe
(86) International application number: PCT/EP2003/013194
(87) International publication number: WO 2004/050601

(56) References cited:
- EP-A- 0 636 602
- US-A- 5 391 775
- US-A- 5 756 782

## Description

### Field of the invention

The present invention relates to a process for the purification of diacerein, which allows to obtain diacerein with a low aloe-emodine content (lower than 100 ppm or, if desired, from 0 to 5 ppm) and is easy to carry out.

### State of the art

Diacerein (1,8-diacetoxy-3-carboxy-antrachinone) is a known compound with antiarthritic activity, obtainable with various processes (see The Merck Index, XIII and., 2979; EP 0 243 698; EP 0 520 414; EP 636 602; PCT EP 00/03691, PCT EP 01/06019), generally through acetylation of aloin (10-glucopyranosyl-1,8-dihydroxy-3-hydroxymethyl-9(10H)-anthracenone; The Merck Index, XIII and., 304) followed by chromic oxydation of the acetyl derivative. This process was disclosed about one hundred years ago by R. Robinson and J. L. Simonsen (Journal of the Chemical Society (Transactions), 1909, 1085-1095). In the paper aloin is referred to by means of its synonim, barbaloin (see The Merck Index, XIII and., 304); a substantially identical process is also disclosed in the aforementioned EP 0 636 602, which also discloses a process for the purification of the resulting crude diacerein, in order to reduce the aloe-emodine (1,8-dihydroxy-3-hydroxymethylantrachinone) content below 70-20 ppm. In fact, even though aloe-emodine is defined as a cathartic compound (The Merck Index, XIII ed., 303), mutagenic properties have also been attributed thereto, even though convincing proofs in this respect have not been given yet. According to the current good manufacturing practices (GPM), pharmaceutical marketed products must contain the lowest possible amount of impurities (this is based on the assumption that substances devoid of a therapeutical effect are noxious, for the mere fact that they are chemicals); in the case of diacerein, numerous attempts have been made to reduce to the minimum the content of the allegedly mutagenic aloin-emodine, for instance by crystallization of crude diacerein from various solvents. For example, the aloe-emodine content can be reduced to 50-100 ppm by crystallising from acetic anhydride in admixture with acetic acid, as disclosed in PCT/EP 00/03691, whereas according to EP 0 636 602 (page 4, lines 25-28) the crystallization of crude diacerein from 2-methoxyethanol or dimethylacetamide yields an aloe-emodine content lower than 70 ppm. This patent also reports an aloe-emodine content lower than 20 ppm (page 5, lines 7-9), which can be obtained through the process described on page 4, page 5 (lines 1-6) and in the examples, as summarised hereinbelow.

### Procedure according to example 1 of EP 0 636 602:

a) drying crude diacerein from the oxydation step until the water content is lower than 1%;
b) salification with triethylamine in methylene chloride and filtration of the solution;
c) acidification of the filtrate with aqueous acetic acid and further acidification with 32% hydrochloric acid;
d) centrifugation and drying at 70-80°C until loss-on-drying is lower than 0.5%;
e) crystallization from 2-methoxyethanol by heating under reflux for three hours, cooling to +5°C, centrifugation of the precipitate and drying until loss on drying is lower than 0.5%;
f) crystallization from anhydrous dimethylacetamide by heating at 110°C for 30 minutes, cooling to 0°C, centrifugation, re-suspension of the solid in deionized water, further centrifugation, washing with water for six times and final drying.

The overall yield of steps b) to f) is 74%.

### Procedure according to example 2 of EP 0 636 602:

a) see above, example 1;
b) crystallization from dimethylacetamide and acetic anhydride by heating at 100°C for 15 minutes, hot-filtration, cooling to 0-2°C, centrifugation and drying;
c) second crystallization from dimethylacetamide, as described in b);
d) third crystallization from dimethylacetamide, as described in b);
e) final purification by heating in refluxing ethyl alcohol for one hour, cooling to 0-2°C, centrifugation, washing with deionized water to remove the majority of the alcohol and final drying.

The overall yield of steps b) to e) is 65%.

It is not clear if emodine levels lower than 70 ppm (page 4, line 28) or 20 ppm (page 5, line 9) can be obtained according to example 1 or according to example 2, respectively, or viceversa. Nevertheless, it is evident that both methods are troublesome and provoke a remarkable decrease in the yield of pure diacerein with respect to crude diacerein. Moreover, they are highly expensive in terms of solvents, equipments and time, not only to obtain pure diacerein but also to recover the diacerein that is lost thoughout the various steps and the high volumes of methoxyethanol and dimethylacetamide.

EP 520 414 and EP 554 880 teach to obtain diacerein with very low aloe-emodine contents by liquid-liquid separation. The processes are carried out, respectively, on diacerein and on rein-9-antron-8-glycoside, which is in turn obtained from Senna and subsequently transformed into diacerein. In both cases the yields are high, but the liquid-liquid separation procedure requires the use of a particular apparatus ("Mixer-Settler-Apparatus" with 60 mixing-separation units) and of thirty volumes of organic phase per volume of mixture from which aloe-emodine is to be extracted, which is considerably diluted. Moreover, the whole process (from the starting material to pure diacerein) comprises at least six steps.

### Detailed disclosure of the invention

It has now been found that diacerein with a very low aloe-emodine content can be obtained by salifying crude diacerein (prepared from aloin according to the process described by Robinson e Simonsen) with a weak base, subjecting an organic-aqueous solution of the salt to discontinuous or continuous extraction in a water-immiscible or sparingly miscible solvent and precipitating pure diacerein by acidification.

In the present application the term "aloe-emodine" means either "aloe-emodine" as such, or aloe-emodine in admixture with the corresponding mono, di- and/or tri-acetyl derivatives which might also be present in crude diacerein.

The weak base is preferably a weak organic base, more preferably selected from the group consisting of trimethylamine, triethylamine, tripropylamine, tributylamine, pyrrolidine and mixtures thereof. The molar ratio diacereine/organic base ranges from 1:1 to 1:1.15; diacereine and the organic base are preferably in a substantially stoichiometric ratio.

The aqueous-organic solvent is a mixture of water and a solvent selected from the group consisting of acetone, methyl ethyl ketone, ethanol, propanol, isopropanol, other water-soluble solvents and mixtures thereof. The volume ratio of water to solvent (or solvent mixture) ranges from 20:80 to 80:20, preferably from 60:40 to 40:60, depending on the organic solvent.

Suitable water-immiscible or sparingly miscible solvents are acetates and propionates of lower alcohols, aromatic hydrocarbons, aliphatic or aromatic halohydrocarbons and mixtures thereof. Particularly preferred are acetates of lower alcohols, in particular ethyl and butyl acetate, toluene and xylene. The number of extraction steps, which depends on the solvent and on the volume ratio solvent/diacerein salt solution, can be easily determined by the person skilled in the art with preliminary tests, depending on the aloe-emodine content in the final product. The same applies when the extraction is carried out in a continuous extractor.

The determination of the aloe-emodine content is carried out by HPLC with the external standard method. The reference solution is prepared by accurately weighing 40 mg of diacerein, 10 mg of aloe-emodine and 20 mg of rein in 50 ml of dimethylacetamide; 1 ml of the solution is diluted to 100 ml with the mobile phase (see below). The chromatographic conditions are as follows:
- Apparatus: Perkin Elmer chromatograph Series 200 pump fitted with a diode array or a similar apparatus;
- Column: Lichrosphere 100 RP-18 (5(m), 250 mm x 4 mm I.D. or equivalent thereof;
- Mobile phase: acetic acid (pH = 2.7)/acetonitrile (53/47) solution;
- Flow: 0.8 ml/min;
- Detection wavelength: 254 nm;
- Injection volume: 20 µl;
- Analysis time: 35 min;
- Integration parameters: bunching factor 3 - threshold area 50 - noise 100 (these values are only indicative and should be adjusted case by case to optimize integration);
In these conditions the following retention times are obtained:
- Diacerein: 7.2;
- Aloe-emodine: 11.5;
- Rein: 12.2.

The reference solution (20 µl) is injected and eluted. If the peak resolution between aloe-emodine and rein in the chromatogram is lower than 1.4, the column is washed with water (15 min; 1 ml/min flow), a 50/50 water/acetonitrile mixture (15 min; 1 ml/min flow), acetonitrile (15 min a flow 1 ml/min) and the test is repeated.

Purified diacerein is recovered from the salt solution by acidification, for example with hydrochloric or phosphoric acid; after centrifugation, washing with water and drying, diacerein is crystallized from acetic acid/acetic anhydride, as disclosed in PCT/EP 00/03691 and in PCT/EP 01/06019.

The following examples illustrate the invention in more detail.

### Example 1

A suspension of 15.6 kg of crude diacerein, containing about 500 ppm of aloe-emodine, in a mixture of 80 1 of acetone and 80 1 of water is added with 4.27 kg of trietylamine. The resulting solution is extracted with four aliquots of butyl acetate (100 1 each); the organic phases are pooled and butyl acetate is recovered and recycled to the process. The diacerein salt solution is acidified with diluted HCl; precipitated diacerein is centrifuged, thoroughly washed with water and dried to afford 14.8 kg of diacerein with an aloe-emodine content not higher than 2 parts per million. Crystallisation from acetic anhydride/acetic acid is subsequently carried out according to what reported above.

### Example 2

The same procedure as example 1 is followed, limiting the number of the extractions to three (final aloe-emodine content ∼ 45 ppm) or two (final aloe-emodine content = 85 ppm).

### Example 3

The same procedure as example 1 is followed, using toluene as the extraction solvent. After five subsequent extractions diacerein contains no more than 3 ppm of aloe-emodine (about 33 ppm after four extractions).

### Example 4

The same procedure as example 1 is followed, using methylene chloride as the extraction solvent. After four extractions diacerein with aloe-emodine content of about 3 ppm is obtained.

### Example 5

The same procedure as the previous examples is followed, using tributylamine as the base and butyl acetate as the extraction solvent. The aloe-emodine content is lower than 2 ppm.

### Example 6

A solution of 100 grams of crude diacerein (aloe-emodine content of about 500 ppm) in 500 ml of methyl ethyl ketone, 500 ml of water, 27.5 grams of triethylamine and 50 ml of methylene chloride is loaded in a Soxhlet apparatus suitable for liquid-liquid extraction. 1 Litre of methylene chloride is loaded in the round-bottom flask for the extraction solvent and heated up to reflux temperature. Extraction is continued for about one hour (the extraction solvent, being denser than the aqueous acetone phase, passes up through it and overflows from the body of the Soxhlet, siphoning over to the flask containing methylene chloride, thus removing the extracted aloe-emodine), thereafter the extraction solvent is then replaced with 500 ml of fresh methylene chloride and extraction is continued for another hour. The water-acetone solution of the diacerein salt is allowed to stand, then separated from the methylene chloride phase (containing emodine traces) and acidified to pH 1 with diluted hydrochloric acid to precipitate diacerein. After filtration and drying under vacuum at 60°C, diacerein (94 grams) contains less than 4 parts per million of aloe-emodine. The extraction can also be interrupted and addition of fresh methylene chloride can be avoided when aloe-emodine levels sufficiently low for the intended use are reached.

Highly pure diacerein can be obtained by crystallization from acetic acid/acetic anhydride (70/9 v/v).

## Claims

1. A process for obtaining diacerein with a content of aloe-emodine and mono-, di- and tri-acetyl derivatives thereof lower than 100 parts per million, **characterized in that** an aqueous-organic solution of a diacerein salt with a weak base is extracted with a water immiscible or sparingly water-miscible solvent.

2. A process as claimed in claim 1 wherein the content of aloe-emodine and mono-, di- and tri-acetyl derivatives thereof ranges from 0 to 5 parts per million.

3. A process as claimed in claim 1 or 2, **characterized in that** the weak base is an organic base selected from the group consisting of trimethylamine, triethylamine, pyrrolidine, ter-butylamine, other weak organic bases and mixtures thereof.

4. Process as claimed in any one of claims 1 to 3, **characterized in that** the organic-aqueous solvent is a mixture of water and a water-miscible solvent selected from the group consisting of acetone, methyl ethyl ketone, methanol, ethanol, propanol, isopropanol, other and mixtures thereof.

5. A process as claimed in claim 4, **characterized in that** the volume ratio of water to water-miscible organic solvent ranges from 80:20 to 20:80.

6. A process as claimed in claim 5, **characterized in that** the volume ratio ranges from 60:40 to 40:60.

7. A process as claimed in any one of claims 1 to 6, **characterized in that** the extraction solvent is selected from the group consisting of acetates, propionates or butyrates of C₂-C₄ alcohols, aromatic hydrocarbons, aliphatic or aromatic halohydrocarbons and mixtures thereof.

8. A process as claimed in claim 7, **characterized in that** the solvent is selected from the group consisting of ethyl and butyl acetate, toluene, xylene, methylene chloride and mixtures thereof.

9. A process according to any one of claims 1 to 8, **characterized in that** the extraction is carried out discontinuously.

10. A process according to any one of claims 1 to 7, **characterized in that** the extraction is carried out continuously.

## Patentansprüche

1. Verfahren zum Erhalten von Diacerein mit einem Gehalt an Aloe-Emodin und Mono-, Di- und Triacetyl-Derivaten davon von weniger als 100 Teilen pro Million, **dadurch gekennzeichnet, dass** eine wässrige organische Lösung eines Diacereinsalzes mit einer schwachen Base mit einem wasserunmischbaren oder schwer wassermischbaren Lösungsmittel extrahiert wird.

2. Verfahren, wie in Anspruch 1 beansprucht, wobei der Gehalt an Aloe-Emodin und Mono-, Di- und Triacetyl-Derivaten davon von 0 bis 5 Teilen pro Million reicht.

3. Verfahren, wie in Anspruch 1 oder 2 beansprucht, **dadurch gekennzeichnet, dass** die schwache Base eine organische Base ist, ausgewählt aus der Gruppe, bestehend aus Trimethylamin, Triethylamin, Pyrrolidin, ter-Butylamin, anderen schwachen organischen Basen und Gemischen davon.

4. Verfahren, wie in einem der Ansprüche 1 bis 3 beansprucht, **dadurch gekennzeichnet, dass** das organische wässrige Lösungsmittel ein Gemisch von Wasser und einem wassermischbaren Lösungsmittel ist, ausgewählt aus der Gruppe, bestehend aus Aceton, Methylethylketon, Methanol, Ethanol, Propanol, Isopropanol, anderen und Gemischen davon.

5. Verfahren, wie in Anspruch 4 beansprucht, **dadurch gekennzeichnet, dass** das Volumenverhältnis von Wasser zu wassermischbarem organischem Lösungsmittel von 80:20 bis 20:80 reicht.

6. Verfahren, wie in Anspruch 5 beansprucht, **dadurch gekennzeichnet, dass** das Volumenverhältnis von 60:40 bis 40:60 reicht.

7. Verfahren, wie in einem der Ansprüche 1 bis 6 beansprucht, **dadurch gekennzeichnet, dass** das Extraktionslösungsmittel ausgewählt wird aus der Gruppe, bestehend aus Acetaten, Propionaten oder Butyraten von C₂-C₄-Alkoholen, aromatischen Kohlenwasserstoffen, aliphatischen oder aromatischen Halogenkohlenwasserstoffen und Gemischen davon.

8. Verfahren, wie in Anspruch 7 beansprucht, **dadurch gekennzeichnet, dass** das Lösungsmittel ausgewählt wird aus der Gruppe, bestehend aus Ethyl- und Butylacetat, Toluol, Xylol, Methylenchlorid und Gemischen davon.

9. Verfahren gemäß einem der Ansprüche 1 bis 8, **dadurch gekennzeichnet, dass** die Extraktion diskontinuierlich durchgeführt wird.

10. Verfahren gemäß einem der Ansprüche 1 bis 7, **dadurch gekennzeichnet, dass** die Extraktion kontinuierlich durchgeführt wird.

## Revendications

1. Procédé pour obtenir de la diacéréine ayant une teneur d'aloé-émodine et de dérivés mono-, di- et tri-acétylés de celle-ci inférieure à 100 parties par million, **caractérisé en ce qu'**une solution organique aqueuse de sel de diacéréine avec une base faible est extraite avec un solvant non miscible avec l'eau ou peu miscible avec l'eau.

2. Procédé comme revendiqué dans la revendication 1, dans lequel la teneur d'aloé-émodine et de dérivés mono-, di- et tri-acétylés de celle-ci se situe entre 0 et 5 parties par million.

3. Procédé comme revendiqué dans la revendication 1 ou 2, **caractérisé en ce que** la base faible est une base organique choisie dans le groupe constitué par la triméthylamine, la triéthylamine, la pyrrolidine, la ter-butylamine, d'autres bases organiques faibles et des mélanges de celles-ci.

4. Procédé comme revendiqué dans l'une quelconque des revendications 1 à 3, **caractérisé en ce que** le solvant organique aqueux est un mélange d'eau et d'un solvant miscible avec l'eau choisi dans le groupe constitué par l'acétone, la méthyléthylcétone, le méthanol, l'éthanol, le propanol, l'isopropanol, un autre solvant et des mélanges de ceux-ci.

5. Procédé comme revendiqué dans la revendication 4, **caractérisé en ce que** le rapport en volume de l'eau au solvant organique miscible avec l'eau se situe entre 80:20 et 20:80.

6. Procédé comme revendiqué dans la revendication 5, **caractérisé en ce que** le rapport en volume se situe entre 60:40 et 40:60.

7. Procédé comme revendiqué dans l'une quelconque des revendications 1 à 6, **caractérisé en ce que** le solvant d'extraction est choisi dans le groupe constitué par des acétates, des propionates ou des butyrates d'alcools en C₂-C₄, des hydrocarbures aromatiques, des halogénohydrocarbures aliphatiques ou aromatiques et des mélanges de ceux-ci.

8. Procédé comme revendiqué dans la revendication 7, **caractérisé en ce que** le solvant est choisi dans le groupe constitué par l'acétate d'éthyle et l'acétate de butyle, le toluène, le xylène, le chlorure de méthylène et des mélanges de ceux-ci.

9. Procédé selon l'une quelconque des revendications 1 à 8, **caractérisé en ce que** l'extraction est effectuée de manière discontinue.

10. Procédé selon l'une quelconque des revendications 1 à 7, **caractérisé en ce que** l'extraction est effectuée en continu.
